# EUROPÄISCHE PATENTSCHRIFT

(11) **EP 1 875 857 B1**
(45) Veröffentlichungstag und Bekanntmachung des Hinweises auf die Patenterteilung: **11.11.2009**
(21) Anmeldenummer: 06116853.0
(22) Anmeldetag: 07.07.2006
(51) Int. Cl.: A61B 3/15

(54) **Ophthalmoskop**
Ophthalmoscope
Ophthalmoscope

(43) Veröffentlichungstag der Anmeldung: 09.01.2008
(73) Patentinhaber: OD-OS GmbH, 14513 Teltow (DE)
(72) Erfinder: TEIWES, Winfried, 14532, Kleinmachnow (DE); HUPPERTZ, Michael, 14163, Berlin (DE); LIESFELD, Ben, 14469, Potsdam (DE)
(74) Vertreter: Betten & Resch

(56) Entgegenhaltungen:
- WO-A-02/094088
- US-B2- 6 758 564

## Beschreibung

Die vorliegende Erfindung betrifft ein Ophthalmoskop zur Untersuchung eines Auges eines Patienten.

Derartige optische Geräte werden insbesondere zur Beobachtung des Augenhintergrunds des Patienten eingesetzt, beispielsweise für Netzhautuntersuchungen. Ein Ophthalmoskop muß hochauflösende Farb- bzw. Schwarzweißbilder in kontinuierlicher Abfolge liefern, um zur Diagnose des Auges ebenso wie bei der Durchführung und Dokumentation von therapeutischen Eingriffen eingesetzt werden zu können. Hierbei stellt die Abbildung des Augenhintergrunds, beispielsweise der Netzhaut, eine optische Herausforderung dar, denn sowohl die Beleuchtung als auch die Beobachtung des Augenhintergrunds müssen durch eine verhältnismäßig kleine Eintrittspupille des Auges durchgeführt werden. Zudem weist der Augenhintergrund in der Regel nur eine schwache Reflektivität auf, die für rote Farbanteile dominiert, so dass kontrastreiche Farbbilder vom Augenhintergrund in der Regel nur mit einer Lichtquelle mit starken Blau- und Grünanteilen erzeugt werden können.

Die optische Untersuchung des Augenhintergrunds mit Hilfe eines Beleuchtungsstrahls, der nach Reflexion beispielsweise an der Netzhaut als Beobachtungsstrahl untersucht wird, wird zudem durch unerwünschte weitere Reflexionen an den Grenzflächen der Kornea sowie durch unerwünschte Lichtstreuung beispielsweise an Glaskörpertrübungen erschwert. Im nachfolgenden werden all diese störenden Lichtstrahlen, die im Beobachtungsstrahl enthalten sein können, jedoch nicht auf die gewünschte Reflexion des Beleuchtungsstrahls am zu untersuchenden Teil des Auges zurückzuführen sind, zusammenfassend als "Streulicht" bezeichnet.

Zur Lösung dieses Streulichtproblems offenbart die EP 1389943 B1 ein Ophthalmoskop gemäß dem Oberbergriff von Anspruch 1.

Als Beleuchtungsvorrichtung wird hierbei insbesondere eine Halogenlampe eingesetzt, deren Licht mittels eines Kondensors kollimiert und über die Beleuchtungs-Abbildungsoptik auf den Augenhintergrund des Patienten fokussiert wird. Der am Augenhintergrund reflektierte Beobachtungsstrahl wird über die Beobachtungs-Abbildungsoptik auf eine Bildebene abgebildet, in der sich ein CCD-Sensor als Beobachtungsvorrichtung befindet.

Die Mittel zum Scannen des Beleuchtungsstrahls über das Auge sind bei diesem gattungsgemäßen Ophthalmoskop durch eine Schlitzblende gebildet, die vor der Halogenlampe in dem vom Kondensor kollimierten Beleuchtungsstrahl oszilliert. Diese Blende ist aus einem lichtundurchlässigen Material, beispielsweise einem Flachblechmaterial, gefertigt und läßt nur einen durch die Größe der Schlitzblende definierten linienförmigen Ausschnitt aus dem Beleuchtungsstrahl durch, der aufgrund der Oszillation der Schlitzblende somit ebenfalls mit dieser Oszillationsfrequenz über das Auge des Patienten hin- und her gescannt wird.

Auch die Mittel zum Ausblenden von Streulicht aus dem Beobachtungsstrahl sind bei diesem gattungsgemäßen Ophthalmoskop durch eine mechanisch oszillierende Schlitzblende gebildet. Insbesondere schlägt die EP 1389943 B1 vor, die vor der Halogenlampe oszillierende Schlitzblende und die vor dem CCD-Sensor oszillierende Schlitzblende, die aus Abbildungsgründen ohnehin miteinander synchronisiert werden müssen, als Blendenspaltpaar in einem gemeinsamen Flachblech auszubilden.

In der Praxis hat sich hierbei gezeigt, dass insbesondere die Wahl eines vor dem Sensor hin- und her schwingenden Blechs mit einer Schlitzblende zu Problemen führt. Denn einerseits lassen sich die Schwingungen des mechanischen Oszillators für das Blech nur ungenügend vom Gehäuse des Gerätes entkoppeln, so dass allgemein Schwierigkeiten in der Handhabung des gattungsgemäßen Ophthalmoskops und insbesondere Beeinträchtigungen in der Bildschärfe aufgrund eines Mitvibrierens des Sensors auftreten.

Andererseits sollte zur Erzielung einer möglichst kompakten Ausführung des Ophthalmoskops die Blende im Beobachtungsstrahl möglichst exakt in der Bildebene der Beobachtungsvorrichtung und somit auf dem Sensor liegen. Es muß jedoch ein gewisser Mindestabstand des mechanisch hin- und her schwingenden Blechs vom Sensor eingehalten werden, was unweigerlich zu einer schlechteren Unterdrückung störenden Streulichts führt.

Es ist daher Aufgabe der Erfindung, ein gattungsgemäßes Ophthalmoskop derart weiterzuentwickeln, dass die Zahl mechanisch schwingender Bauteile verringert wird, insbesondere in der Nähe der Beobachtungsvorrichtung.

Erfindungsgemäß wird diese Aufgabe durch ein Ophthalmoskop nach Anspruch 1 gelöst.

Derartige elektronische Sensoren, insbesondere CMOS-Sensoren, sind im Stand der Technik an sich bekannt, vgl. beispielsweise die WO 99/05853. Der Einsatz eines derartigen elektronischen Sensors bei einem Ophthalmoskop erlaubt es nun erstmals, die im Stand der Technik benutzte mechanische Blende gleichsam durch eine elektronische Blende ("rolling shutter") zu ersetzen. Denn mit Hilfe der elektronischen Steuerschaltung können alle Pixelzeilen eines derartigen Sensors gezielt aktiviert, deaktiviert und ausgelesen werden. Im aktivierten Zustand eines Pixels führt Lichteinfall zur kontinuierlichen Erzeugung von Ladungen, die bei einem CMOS-Sensor über eine dem jeweiligen Pixel zugeordnete Verstärkerelektronik in ein Spannungssignal umgesetzt werden. Im deaktivierten Zustand hingegen unterbleibt ein derartiger Spannungsaufbau, das Pixel oder auch die gesamte Pixelzeile ist gleichsam "abgeschaltet".

Durch gezieltes Aktivieren wenigstens einer Pixelzeile in einem bestimmten Bereich des Felds von fotosensitiven Pixeln und gleichzeitiges Deaktivieren aller übrigen Pixelzeilen kann somit auf elektronische Weise das gleiche Ergebnis wie mit Hilfe einer mechanischen Schlitzblende erzielt werden. Beim erfindungsgemäßen Ophthalmoskop kann somit Streulicht aus dem Beobachtungsstrahl ausgeblendet werden, ohne dass hierfür ein mechanisch schwingendes Blech mit einer Schlitzblende vor der Beobachtungsvorrichtung erforderlich ist. Zudem befindet sich die erfindungsgemäß vorgesehene elektronische Blende im Gegensatz zur mechanischen Blende beim gattungsgemäßen Ophthalmoskop nicht vor, sondern in der Bildebene der Beobachtungsvorrichtung, was die Streulichtunterdrückung verbessert.

Zweckmäßigerweise ist vorgesehen, dass die elektronische Steuerschaltung dazu ausgelegt ist, jeweils eine einzelne Pixelzeile auszulesen. Auf diese Weise läßt sich eine äußerst feine Auflösung des elektronischen Sensors erzielen, die bei typischen CMOS-Sensoren einer Höhe von ca. 5µm entspricht. Es wäre jedoch grundsätzlich auch vorstellbar, mit Hilfe der elektronischen Steuerschaltung mehrere Pixelzeilen, insbesondere benachbarte Pixelzeilen, zusammenzufassen, d.h. stets gemeinsam zu aktivieren, zu deaktivieren oder auszulesen, und somit unter Verschlechterung der Auflösung die Lichtausbeute zu verbessern.

Zweckmäßigerweise ist die elektronische Steuerschaltung dazu ausgelegt, jeweils nach Ablauf einer einstellbaren Zeitverzögerung (Δt) die aktuell auszulesende Pixelzeile zu ändern, insbesondere von einer Pixelzeile zu einer benachbarten Pixelzeile. In der bevorzugten Ausführungsvariante, bei der unmittelbar benachbarte Pixelzeilen nacheinander ausgelesen werden, benötigt die elektronische Steuerschaltung somit für einen vollständigen Durchlauf, d.h. ein vollständiges Auslesen des elektronischen Sensors mit N Pixelzeilen, insgesamt eine Zeitdauer T=NxΔt. Hierbei kann der Paramter N, der die Zahl der Pixelzeilen des Sensors beschreibt, ausgehend von einer durch die Bauart des Sensors vorgegebenen Obergrenze durch das oben beschriebene elektronische Zusammenfassen von benachbarten Pixelzeilen reduziert werden, während gleichzeitig der Zeitverzögerungsparameter Δt vom Bediener des erfindungsgemäßen Ophthalmoskops frei einstellbar ist. Auf diese Weise kann die für einen vollständigen Auslese-Durchlauf benötigte Gesamtzeitdauer eingestellt werden, entsprechend der Einstellung der Schwingungsperiodendauer bzw. der Schwingungsfrequenz der beim gattungsgemäßen Ophthalmoskop vorgesehenen mechanischen Schlitzblende.

In einer bevorzugten Ausführungsform der Erfindung ist die elektronische Steuerschaltung ferner dazu ausgelegt, jede Pixelzeile während einer einstellbaren Belichtungszeit (T_{INT}) vor ihrem Auslesen zu aktivieren. Durch Einstellen dieser Belichtungszeit T_{INT} als Vielfaches der Zeitverzögerung Δt läßt sich somit erreichen, dass jede Pixelzeile während eines längeren Zeitraums t_{INT} belichtet wird, während dessen sich ihr die aktuell auszulesende Pixelzeile nähert. Auf diese Weise kann die effektive Breite der elektronischen Spaltblende flexibel eingestellt werden.

Zweckmäßigerweise ist die elektronische Steuerschaltung dazu ausgelegt, den Auslesevorgang infolge eines externen Triggersignals zu starten. Auf diese Weise kann die auch beim erfindungsgemäßen Ophthalmoskop erforderliche Synchronisierung der Blende für die Beobachtungsvorrichtung mit den Mitteln zum Scannen des Beleuchtungsstrahls sichergestellt werden. Beispielsweise kann ein zentraler Steuercomputer für das erfindungsgemäße Ophthalmoskop einerseits mit Hilfe dieses Triggersignals die elektronische Steuerschaltung des elektronischen Sensors und andererseits einen Antrieb für eine mechanische Schlitzblende im Beleuchtungsstrahl kontrollieren.

Grundsätzlich läßt sich mit Hilfe eines derartigen zentralen Steuercomputers eine Synchronisierung der elektronischen Blende im Beobachtungsstrahl mit einer mechanischen Blende im Beleuchtungsstrahl problemlos erzielen. Der zentrale Steuercomputer kann selbstverständlich auch Teil des elektronischen Sensors oder Teil der Beleuchtungsvorrichtung sein.

Um nun jedoch auch im Beleuchtungsstrahl eine mechanisch schwingende Schlitzblende überflüssig zu machen, kann in einer vorteilhaften Weiterbildung des erfindungsgemäßen Ophthalmoskops vorgesehen sein, dass die Mittel zum Scannen des Beleuchtungsstrahls über das Auge einen schwenkbar gelagerten Spiegel mit einer Scanvorrichtung umfassen. Um sicherzustellen, dass der auf den schwenkbar gelagerten Spiegel auftreffende Beleuchtungsstrahl bereits die gewünschte Linienform besitzt, kann beim erfindungsgemäßen Ophthalmoskop entweder vorgesehen sein, dass es ferner eine feste Spaltblende zum Auskoppeln eines linienförmigen Beleuchtungsstrahls umfaßt, oder es ferner eine Linienfokussieroptik zum Fokussieren des Beleuchtungsstrahls in einer Linie umfaßt, wobei in diesem letztgenannten Fall zweckmäßigerweise die Linienfokussieroptik eine Zylinderlinse umfaßt. Die Verwendung eines derartigen schwenkbar gelagerten Spiegels, der mit Hilfe eines Galvanometerantriebs angetrieben und bei einem Ophthalmoskop eingesetzt wird, um einen von einer Linienfokussieroptik gelieferten Beleuchtungsstrahl zu scannen, ist aus der US 6,758,564 B2 grundsätzlich bekannt, auf die insofern verwiesen wird.

Für eine zentrale Kontrolle der wesentlichen Bauteile des erfindungsgemäßen Ophthalmoskops ist zweckmäßigerweise vorgesehen, dass die elektronische Kontrolleinheit dazu ausgelegt ist, die elektronische Steuerschaltung des elektronischen Sensors und die Scanvorrichtung des schwenkbaren Spiegels kontrollieren. Die elektronische Kontrolleinheit kann dann durch Senden des oben besprochenen Triggersignals an die elektronische Steuerschaltung des elektronischen Sensors sicherstellen, dass bei einem beispielsweise sinusförmig schwingenden Spiegel der Auslesevorgang im elektronischen Sensor zu einem geeigneten Zeitpunkt startet, beispielsweise durch ein Auslesen der oberen Pixelzeile des elektronischen Sensors, sobald der schwingende Spiegel seinen oberen Umkehrpunkt erreicht hat.

Bei dieser Weiterbildung des erfindungsgemäßen Ophthalmoskops ist die elektronische Kontrolleinheit dazu ausgelegt , die Scanvorrichtung und die elektronische Steuerschaltung derart zu kontrollieren, dass das Scannen des Beleuchtungsstrahls über das Auge synchron mit der Änderung der aktuell auszulesenden Pixelzeile erfolgt. Beispielsweise kann die elektronische Kontrolleinheit der Scanvorrichtung eine Sägezahntrajektorie vorgeben, die exakt der Bewegung des elektronischen Spalts im elektronischen Sensor entspricht.

Für besonders präzise Untersuchungen des Auges des Patienten mit besonders hoher Ortsauflösung ist vorgesehen, dass die Stärke der Linie des fokussierten Beleuchtungsstrahls der Höhe einer Pixelzeile des elektronischen Sensors entspricht. Man erzielt hierdurch eine konfokale Abbildung des Auges, wodurch sich dreidimensionale Informationen über das beobachtete Objekt gewinnen lassen.

Bei Verwendung der besprochenen Linienfokussieroptik kann die Beleuchtungsvorrichtung zweckmäßigerweise ein Laser sein. Alternativ kann jede andere Form einer im wesentlichen punktförmigen Beleuchtungsvorrichtung eingesetzt werden, beispielsweise ein Ende eines beleuchteten Lichtwellenleiters.

In einer Weiterbildung der Erfindung ist vorgesehen, dass die Beobachtungsvorrichtung zwei Sensoren zur stereoskopischen Untersuchung des Auges umfaßt. Bei einer derartigen Ausführungsform werden die Vorteile der vorliegenden Erfindung besonders deutlich, da für eine stereoskopische Betrachtung mit zwei Beobachtungsvorrichtungen im Stand der Technik insgesamt sogar drei hin- und her oszillierende Schlitzblenden erforderlich sind, auf die erfindungsgemäß weitgehend bzw. bei Verwendung der Linienfokussieroptik sogar vollständig verzichtet werden kann.

Schließlich ist in einer weiteren Ausführungsform der Erfindung vorgesehen, dass sie ferner eine separate Lichtquelle und einen separaten Bildsensor zur Untersuchung des Auges umfaßt. Insbesondere kann hierbei mit Hilfe der Beleuchtungsvorrichtung, der Beleuchtungs-Abbildungsoptik, der Beobachtungs-Abbildungsoptik und der Beobachtungsvorrichtung der Augenhintergrund untersucht werden, beispielsweise die Netzhaut des Auges, während die zusätzliche Abbildungseinheit zur Untersuchung des Augenvordergrunds dient. Dies kann sowohl der medizinischen Untersuchung des Augenvordergrunds selbst dienen, als auch der automatisierten Positionierung des Auges des Patienten, als auch einer Verfolgung von Augenbewegungen (Tracking) des gesamten Auges anhand der Informationen, die man beispielsweise durch Reflexion eines Lichtstrahls am Augenvordergrund erhält. Insbesondere beim Aufsummieren von Augenhintergrund-Bildern bei Angiographie ist es sinnvoll, mittels einer derartigen gleichzeitigen Beobachtung des Augenvordergrunds Augenbewegungen feststellen zu können, die sich zwischen den einzelnen Bildern des Augenhintergrunds ereignet haben.

Bevorzugte Ausführungsformen der Erfindung werden nachfolgend anhand der rein beispielhaften und keineswegs beschränkenden Zeichnungen erläutert werden. Es zeigt:
Figur 1 eine schematische Darstellung eines Ophthalmoskops des Stands der Technik;
Figur 2 eine der Figur 1 ähnliche Darstellung einer ersten Ausführungsform des erfindungsgemäßen Ophthalmoskops;
Figuren 3a bis 3c drei vereinfachte Darstellungen eines Felds von fotosensitiven Pixeln eines elektronischen Sensors zu drei verschiedenen Zeitpunkten während eines Auslesevorgangs;
Figur 4a ein vereinfachtes Zeitdiagramm zur Erläuterung der Bedeutung der Belichtungszeit T_{INT} einer Pixelzeile;
Figur 4b ein vereinfachtes Zeitdiagramm eines Triggersignals zum Starten eines Auslesevorgangs des elektronischen Sensors;
Figur 5 eine schematische Darstellung einer weiteren Ausführungsform des erfindungsgemäßen Ophthalmoskops mit einer Linienfokussieroptik und einem schwenkbar gelagerten Spiegel im Beleuchtungsstrahl;
Figur 6 eine schematische Darstellung einer weiteren Ausführungsform des erfindungsgemäßen Ophthalmoskops mit zwei Beobachtungsstrahlen für stereoskopische Abbildungen; und
Figur 7 eine schematische Darstellung einer weiteren Ausführungsform des erfindungsgemäßen Ophthalmoskops zur gleichzeitigen Untersuchung des Augenvordergrunds.

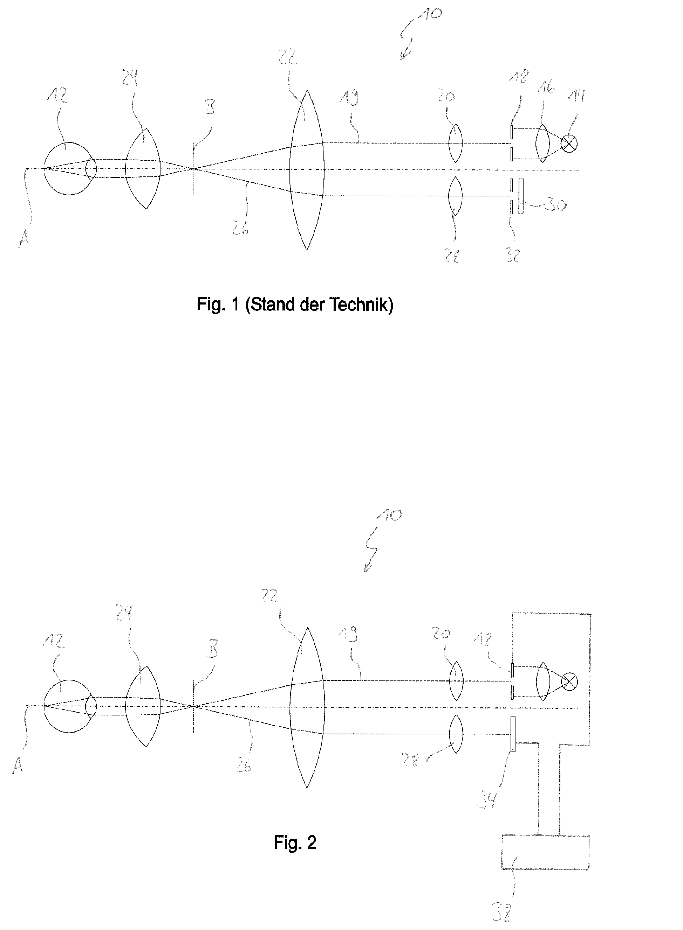

Figur 1 zeigt eine schematische Darstellung eines Ophthalmoskops 10 des Stands der Technik zur Untersuchung eines Auges 12 eines Patienten.

Das von einer Beleuchtungsvorrichtung 14 ausgesandte Licht wird mittels eines Kondensors 16 kollimiert und fällt auf eine Schlitzblende 18, die hin- und her oszilliert, und zwar in der schematischen Darstellung der Figur 1 in der Zeichenebene in einer Richtung senkrecht zur optischen Achse A des Ophthalmoskops 10. Die Schlitzblende 18 ist senkrecht zur Zeichenebene orientiert.

Auf diese Weise wird aus dem flächigen Lichtfleck, zu dem der Kondensor 16 das Licht der Beleuchtungsvorrichtung 14 kollimiert hat, eine Linie ausgekoppelt, deren Länge und Stärke von der Form der Schlitzblende 18 definiert wird, und deren Position von der aktuellen Lage der oszillierenden Schlitzblende abhängt.

Über weitere Linsen 20, 22 wird der Beleuchtungsstrahl 19 in eine Zwischenbildebene B fokussiert, aus der er über über eine ophthalmoskopische Linse 24 auf das Auge 12 abgebildet wird. Die schematische Darstellung der Figur 1 zeigt hierbei eine Abbildung auf den Augenhintergrund, beispielsweise für eine Netzhautuntersuchung.

Der aus der Reflexion des Beleuchtungsstrahls 19 am Augenhintergrund hervorgehende Beobachtungsstrahl bildet den Augenhintergrund über die ophthalmoskopische Linse 24 in die Zwischenbildebene B ab. Dieses Zwischenbild wird mittels der weiteren Linsen 22, 28 auf einen CCD-Sensor 30 fokussiert. Unmittelbar vor Erreichen des CCD-Sensors 30 muß der Beobachtungsstrahl 26 hierbei durch eine weitere Schlitzblende 32 gelangen, die vor dem CCD-Sensor synchron mit der Schlitzblende 18 im Strahlengang des Beleuchtungsstrahls 19 oszilliert.

Auf diese Weise wird mit Hilfe der Schlitzblende 32 sichergestellt, dass im wesentlichen nur der gewünschte Beobachtungsstrahl 26 den CCD-Sensor 30 erreicht, während Streulicht ausgeblendet wird. Derartiges Streulicht kann beispielsweise auf Reflexionen an der Augenvorderseite oder auch auf Streuungen an Glaskörpertrübungen des Auges 12 zurückzuführen sein.

Man erkennt in Figur 1, dass die oszillierende Schlitzblende 32 verhältnismäßig nah am CCD-Sensor 30 angeordnet ist. Dies ist unerlässlich, da mit zunehmendem Abstand zwischen Schlitzblende 32 und CCD-Sensor 30 die Unterdrückung störenden Streulichts verschlechtert wird. Allerdings führt diese Nähe dazu, dass sich Vibrationen von der oszillierenden Schlitzblende 32 auf den CCD-Sensor 30 übertragen können, beispielsweise über ein in den Figuren nicht dargestelltes Gehäuse des Ophthalmoskops 10. Dies führt zu grundsätzlichen Problemen bei der Handhabung des Ophthalmoskops 10, insbesondere verschlechtert sich die Schärfe des vom CCD-Sensor 30 gelieferten Bildes des Auges 12.

Figur 2 zeigt eine der Figur 1 ähnliche schematische Darstellung eines erfindungsgemäßen Ophthalmoskops 10, bei dem diese Probleme vermieden werden. Entsprechende Bauteile tragen in Figur 2 die gleichen Bezugszeichen wie in Figur 1. Das erfindungsgemäße Ophthalmoskop 10 gemäß der in Figur 2 dargestellten Ausführungsform unterscheidet sich von jenem des Stands der Technik dadurch, dass anstelle des CCD-Sensors 30 und der vor ihm hin- und her schwingenden Schlitzblende 32 ein elektronischer Sensor 34 im Beobachtungsstrahl 26 vorgesehen ist, der ein Feld von jeweils zeilenweise aktivierbaren und/oder auslesbaren fotosensitiven Pixeln umfaßt, die mittels einer elektronischen Steuerschaltung einzeln angesteuert werden können. Die elektronische Steuerschaltung ist im elektronischen Sensor 34 angeordnet und in den Figuren nicht dargestellt.

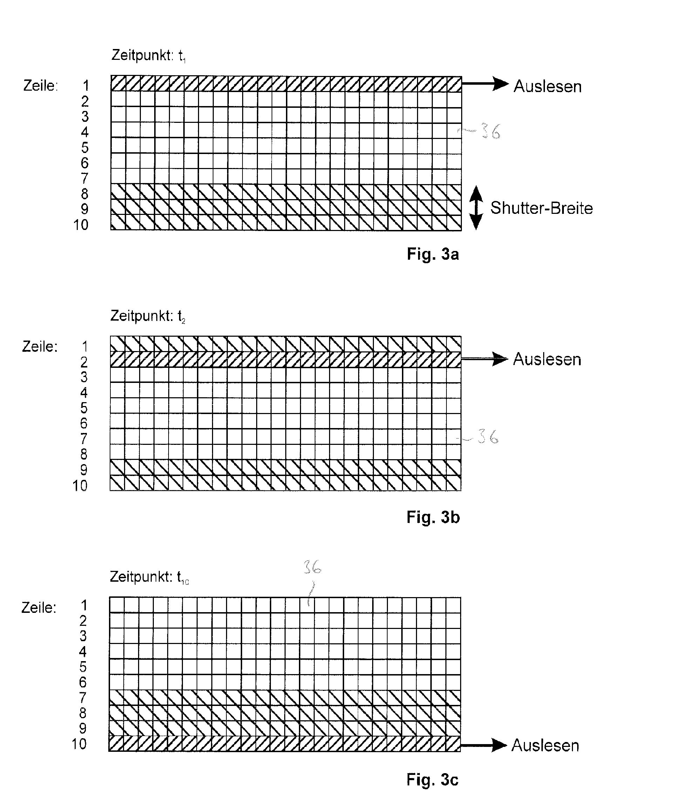

Die Funktionsweise des durch die elektronische Steuerschaltung kontrollierten elektronischen Sensors 34 wird nachfolgend anhand der Figuren 3a bis 3c erläutert werden:

Die Figuren 3a bis 3c zeigen eine schematische Vorderansicht eines Felds von fotosensitiven PixeIn eines vereinfacht dargestellten elektronischen Sensors 34. In der gezeigten vereinfachten Ausführungsform umfaßt dieses Feld von fotosensitiven Pixeln insgesamt 10 Pixelzeilen, die mit 1,2, ..., 10 durchnumeriert sind. Jede Pixelzeile umfaßt in den Figuren 3a bis 3c vierundzwanzig Pixel, die mittels fotosensitiver Elemente abhängig von der einfallenden Lichtmenge Ladungen erzeugen. Jedem Pixel 36 ist eine eigene Verstärkerelektronik zugeordnet, die die im Pixel 36 generierte elektrische Ladung in ein jeweiliges Spannungssignal umwandelt. Die Funktionsweise derartiger elektronischer Sensoren mit sogenannten "aktiven Pixeln", insbesondere CMOS-Sensoren, ist an sich bekannt und wird hier nicht weiter erläutert werden.

Mit Hilfe der im elektronischen Sensor 34 vorgesehenen elektronischen Steuerschaltung kann jede Pixelzeile in einen von drei Betriebszuständen geschaltet werden:
a) Einen deaktivierten Modus, in dem die Pixelzeile trotz Lichteinfalls keine Ladungen integriert und somit kein Spannungssignal erzeugt; in Figur 3a befinden sich die Pixelzeilen 2, 3, ..., 7 in diesem deaktivierten Modus, was durch leere Pixel 36 in diesen Pixelzeilen angedeutet wird.
b) Einen aktiven Modus, in dem Lichteinfall zur Erzeugung eines kontinuierlich ansteigenden Spannungssignals führt; in Figur 3a befinden sich die Pixelzeilen 8, 9 und 10 in diesem Betriebszustand, was durch eine einfache Schraffur dieser Pixelzeilen dargestellt ist.
c) Einen Auslesemodus, in dem eine Pixelzeile ausgelesen wird, d.h. das in einem bestimmten vorangegangenen Belichtungszeitraum aufgebaute Spannungssignal von der elektronischen Steuerschaltung abgerufen wird; die Pixelzeile wird anschließend gelöscht, d.h. das Spannungssignal wieder auf den Wert Null zurückgesetzt. In Figur 3a befindet sich nur die Pixelzeile 1 in diesem Auslesemodus, angedeutet durch die dichte Schraffur.

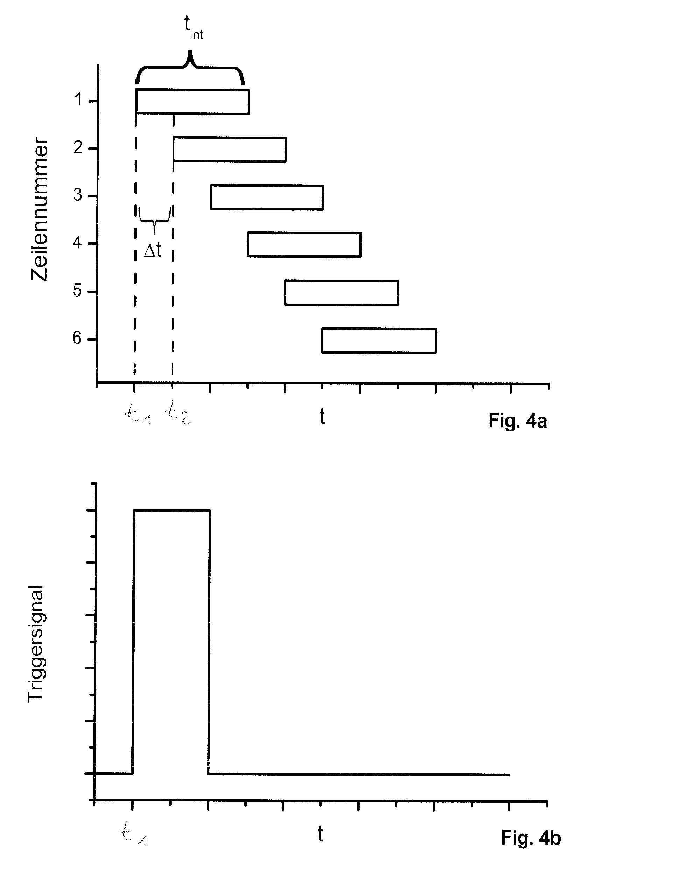

Der durch die elektronische Steuerschaltung kontrollierte Auslesevorgang des elektronischen Sensors 34 wird nachfolgend anhand der Figuren 3a bis 3c, 4a und 4b erläutert werden:

Zu einem in den Zeitdiagrammen der Figuren 4a und 4b gekennzeichneten Startzeitpunkt t₁ empfängt die elektronische Steuerschaltung von einer elektronischen Kontrolleinheit 38, beispielsweise einem zentralen Steuercomputer, ein Triggersignal, beispielsweise ein TTL-Signal. Die ansteigende Flanke des in Figur 4b dargestellten Triggersignals startet einen vollständigen Auslesedurchlauf des elektronischen Sensors 34, indem die Pixelzeile Nummer 1 vom inaktiven Zustand zunächst in den Auslesezustand und anschließend in den aktiven Zustand geschaltet wird, d.h. Lichteinfall führt zum Aufbau eines Spannungssignals in jedem Pixel der ersten Pixelzeile. Die erste Pixelzeile bleibt während einer Belichtungszeitdauer t_{INT} in diesem aktiven Zustand und wird zu einem entsprechenden Zeitpunkt t₁ des nächsten Auslesedurchlaufs von der elektronischen Steuerschaltung ausgelesen.

Nach Ablauf einer einstellbaren Zeitverzögerung Δt nach dem Startzeitpunkt t₁ wird auch die zweite Pixelzeile ausgelesen und in den aktiven Zustand geschaltet, und um eine weitere Zeitverzögerung Δt später auch die dritte Pixelzeile. Bei dem in Figur 4a gezeigten Fall wird t_{INT}=3xΔt gewählt, so dass sich zum Zeitpunkt des Auslesens und Aktivierens der vierten Pixelzeile t₄=t₁+t_{INT}=t₁+3xΔt momentan auch die zweite und die dritte Pixelzeile im aktiven Modus befinden, wie in Figur 3a gezeigt ist, wohingegen die erste Pixelzeile gerade deaktiviert wird.

Nach dem Auslesen der ersten Pixelzeile zum Zeitpunkt t₁ wird diese aktiviert, und eine weitere Zeitverzögerung Δt später, also zu einem Zeitpunkt t₂=t₁+Δt, wird die zweite Pixelzeile ausgelesen, die ab dem Zeitpunkt t₂ des vorhergehenden Durchlaufs während der Zeitdauer t_{INT} belichtet wurde. Auf diese Weise schreitet der Auslesevorgang jeweils in zeitlichen Abständen entsprechend der Zeitverzögerung Δt um eine Pixelzeile des elektronischen Sensors 34 weiter, bei dem in Figuren 3a bis 3c gezeigten Beispiel von Pixelzeile Nummer 1 bis zur Pixelzeile Nummer 10. Die Wahl von t_{INT}=3xΔt führt dazu, dass zum Zeitpunkt des Auslesens der n-ten Pixelzeile drei benachbarte Pixelzeilen aktiv sind, nämlich die Pixelzeilen n-1, n-2 und n-3. Dies definiert die effektive Breite der elektronischen Schlitzblende, die sich in den Figuren 3a bis 3c gleichsam von oben nach unten über das Feld von fotosensitiven Pixeln 36 bewegt. Man beachte, dass die in einer Pixelzeile im Zeitraum t_{INT} aufgebaute Spannung erst im nächsten Durchlauf der elektronischen Auslesung abgefragt wird, wenn diese Pixelzeile in den Auslesemodus geschaltet wird.

Die für einen vollständigen Durchlauf benötigte Gesamtzeit T entspricht dem Produkt aus der Zahl N der Pixelzeilen (im Beispiel der Figuren 3a bis 3c ist N=10) und der Zeitverzögerung Δt, also T=NxΔt. Durch die Wahl der drei Parameter Δt, t_{INT} und N können somit die Bildwiederholungsrate 1/T sowie die effektive Breite der elektronischen Spaltblende flexibler eingestellt werden als die entsprechenden Parameter einer mechanisch oszillierenden Schlitzblende 32 im Stand der Technik gemäß Figur 1.

Die elektronische Kontrolleinheit 38 ist dazu ausgelegt, das in Figur 4b dargestellte Triggersignal zur Synchronisierung der jeweils aktiven Pixelzeilen mit der Bewegung der Schlitzblende 18 im Beleuchtungsstrahl einzusetzen. Zum Zeitpunkt t₁ beispielsweise (siehe Figur 3a) sind die Pixelzeilen 8, 9 und 10 aktiv. Der Zeitpunkt t₁ muß daher derart gewählt sein, dass er einem Zeitpunkt entspricht, an dem die Schlitzblende 18 sich am unteren Umkehrpunkt ihrer Schwingungsbewegung befindet. Die in den Figuren 3a bis 3c und 4a dargestellte Abfolge von Pixelzeilen-Aktivierungsvorgängen von oben (in Figur 3a wird Pixelzeile Nummer 1 ausgelesen und unmittelbar anschließend aktiviert) nach unten (in Figur 3c wird Pixelzeile 10 ausgelesen und unmittelbar anschließend aktiviert) verläuft dann synchron zu einer Bewegung der Schlitzblende 18 von oben nach unten in Figur 2.

Da, wie in den Figuren 3a bis 3c und 4a gezeigt, bei diesem Prinzip des "rolling shutter" gleichsam eine elektronische Blende stets von oben nach unten über das Pixelfeld läuft, dürfen nur solche Bilder des elektronischen Sensors 34 verarbeitet werden, die einer hierzu synchronen Abwärtsbewegung des Beleuchtungsstrahls 19 entsprechen. Diejenigen Schwingungsphasen, in denen sich die Schlitzblende 18 in Figur 2 aufwärts bewegt, sind nicht verwertbar. Aus diesem Grund wird nach einem vollständigen Auslese-Durchlauf des elektronischen Sensors 34, in dem die im vorangegangenen Durchlauf in den einzelnen Pixelzeilen aufgebauten Spannungssignale ausgelesen werden, die Abgabe des nächsten Triggersignals durch die elektronische Kontrolleinheit 38 verzögert, bis die Schlitzblende 18 wieder ihren oberen Umkehrpunkt erreicht hat.

Eine weitere Verbesserung des durch den elektronischen Sensor 34 gelieferten Bilds des Auges 12 läßt sich dadurch erreichen, dass nicht nur der Auslese-Startzeitpunkt t₁ mit dem oberen Umkehrpunkt der schwingenden Schlitzblende 18 synchronisiert wird, sondern auch die Abwärtsbewegung der elektronischen Blende in den Figuren 3a bis 3c auf die entsprechende Abwärtsbewegung der Schlitzblende 18 im Beleuchtungsstrahl 19 abgestimmt wird. Wenn beispielsweise die Schlitzblende 18 sinusförmig im Beleuchtungsstrahl 19 hin- und her schwingt, ist ihre Geschwindigkeit, mit der sie durch den Beleuchtungsstrahl 19 fährt, am oberen und am unteren Umkehrpunkt der Schwingung jeweils gleich Null, während sie in der Mitte zwischen den beiden Umkehrpunkten ein Maximum erreicht. Dies kann im elektronischen Sensor 34 dadurch berücksichtigt werden, dass die Zeitverzögerung Δt abweichend von Figur 4a nicht konstant gewählt wird, sondern als Funktion der jeweiligen Pixelzeile an den oberen und unteren Rändern des Pixelfelds (beispielsweise im Bereich der Pixelzeilen 1 und 10) größer gewählt wird als in der Mitte des Pixelfelds (in der Nähe der Pixelzeilen 5 und 6).

Dieser zusätzliche elektronische Aufwand, der sich ergibt, wenn man die Bewegung des "rolling shutter" des elektronischen Sensors 34 an die Bewegung der mechanischen Schlitzblende 18 anpassen möchte, läßt sich vermeiden, wenn man in umgekehrter Weise den in den Figuren 3a bis 3c, 4a und 4b dargestellten zeitlichen Ablauf der elektronischen Blende beibehält und die Bewegung der mechanischen Schlitzblende hieran anpaßt. Beispielsweise kann die elektronische Kontrolleinheit 38 einen in den Figuren nicht dargestellten Antriebsmechanismus der Schlitzblende 18 derart kontrollieren, dass die Schlitzblende 18 eine Art "Sägezahntrajektorie" durchläuft, also mit konstanter Geschwindigkeit von ihrem oberen Umkehrpunkt zu ihrem unteren Umkehrpunkt bewegt wird und anschließend deutlich schneller an ihren oberen Umkehrpunkt zurückgestellt wird.

Je nach gewünschter Bildwiederholungsfrequenz kann sich eine derartige sägezahnförmige Bewegung eines ausgedehnten Blechs, in dem Schlitzblende 18 vorgesehen ist, jedoch als schwierig erweisen.

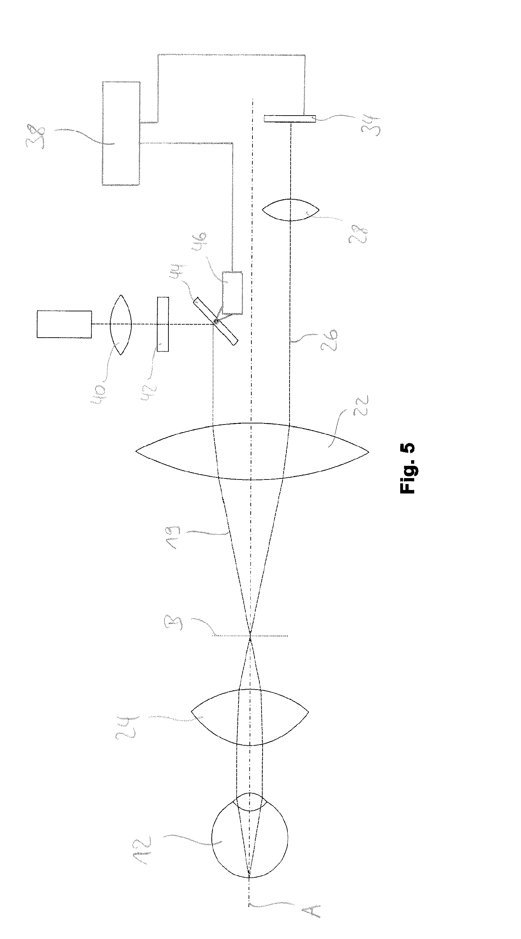

In einer in Figur 5 dargestellten bevorzugten Ausführungsform des erfindungsgemäßen Ophthalmoskops 10 ist daher in Form einer Linienfokussieroptik auch die Erzeugung des Beleuchtungsstrahls 19 gegenüber dem Stand der Technik gemäß Figur 1 abgeändert, um die Synchronisierung mit der elektronischen Blende im elektronischen Sensor 34 zu erleichtern:

In Figur 5 sind Komponenten, die jenen der in Figur 2 dargestellten Ausführungsform entsprechen, mit gleichen Bezugszeichen bezeichnet. Insbesondere wird auch bei der bevorzugten Ausführungsform gemäß Figur 5 ein elektronischer Sensor 34 mit den oben beschriebenen Eigenschaften zur Erfassung des Beobachtungsstrahls verwendet.

In dieser Ausführungsform jedoch wird als Beleuchtungsvorrichtung 14 eine punktförmige Lichtquelle, z.B. ein Laser oder das Ende eines Lichtwellenleiters eingesetzt. Das von ihm ausgehende Licht wird durch einen Kondensor 40 in ein paralleles Strahlenbündel überführt und auf eine Zylinderlinse 42 gerichtet. In Strahlrichtung hinter der Zylinderlinse 42 ist ein schwenkbar gelagerter Spiegel 44 angeordnet, der den Beleuchtungsstrahl 19 annähernd parallel zur optischen Achse A in Figur 5 nach links in Richtung auf die Linse 22 reflektiert, welche sich in der fokussierenden Ebene der Zylinderlinse 42 befindet. Die Zylinderlinse 42 ist hierbei derart positioniert, dass der von ihr erzeugte Linienfokus senkrecht zur Zeichenebene der Figur 5 orientiert ist.

Der Spiegel 44 ist schwenkbar an einer Scanvorrichtung 46 gelagert, die von der elektronischen Kontrolleinheit 38 gesteuert wird, welche gleichzeitig die elektronische Steuerschaltung des elektronischen Sensors 34 kontrolliert. Die elektronische Kontrolleinheit 38 ist dazu ausgelegt, die Scanvorrichtung 46 derart zu kontrollieren, dass sie mittels einer Drehung des Spiegels 44 in der Zeichenebene der Figur 5 den Beleuchtungsstrahl 19 synchron zur Bewegung der elektronischen Blende im elektronischen Sensor 34 über das Auge 12 scannt. Bei dem in den Figuren 3a bis 3c gezeigten kontinuierlichen linearen Abwärtsverlauf der elektronischen Blende kann der Spiegel 44 eine Sägezahntrajektorie durchlaufen, indem er beispielsweise ausgehend von der in Figur 5 gezeigten Stellung schrittweise entgegen dem Uhrzeigersinn gedreht wird, und schließlich, sobald die elektronische Blende im elektronischen Sensor 34 die untere Pixelzeile erreicht hat, beispielsweise die Pixelzeile Nummer 10 in Figur 3c, schlagartig im Uhrzeigersinn in seine Ausgangsposition gemäß Figur 5 zurückgestellt wird.

Ein derartiger schwenkbar gelagerter Spiegel weist in der Praxis ein sehr geringes Trägheitsmoment auf, so dass er nicht nur bei hohen Frequenzen, sondern generell in einem großen Frequenzbereich eingesetzt werden kann. Die Kombination eines derartigen Spiegels mit einem "rolling shutter" gemäß Figur 5, die somit eine freie Wahl der Bildwiederholrate und eine externe Synchronisation mit Hilfe der elektronischen Kontrolleinheit 38 ermöglicht, erlaubt daher eine optimale Anpassung der Beleuchtung des Auges 12 an die jeweils gewünschte Beobachtung. Durch die geringe Trägheit des Spiegels 44 werden Vibrationen im erfindungsgemäßen Ophthalmoskop 10 nahezu vollständig vermieden.

Der Einsatz einer kohärenten Lichtquelle, beispielsweise eines Lasers, erlaubt die Erzeugung eines sehr schmalen Beleuchtungsspalts. Wählt man gleichzeitig eine minimale elektronische Spaltbreite von einer einzigen Pixelzeile entprechend einer Höhe eines Pixel 36 von ca. 5µm, so kann man eine senkrecht zur Spaltausdehnung konfokale Abbildung erzielen und somit dreidimensionale Untersuchungen des Auges 12 vornehmen.

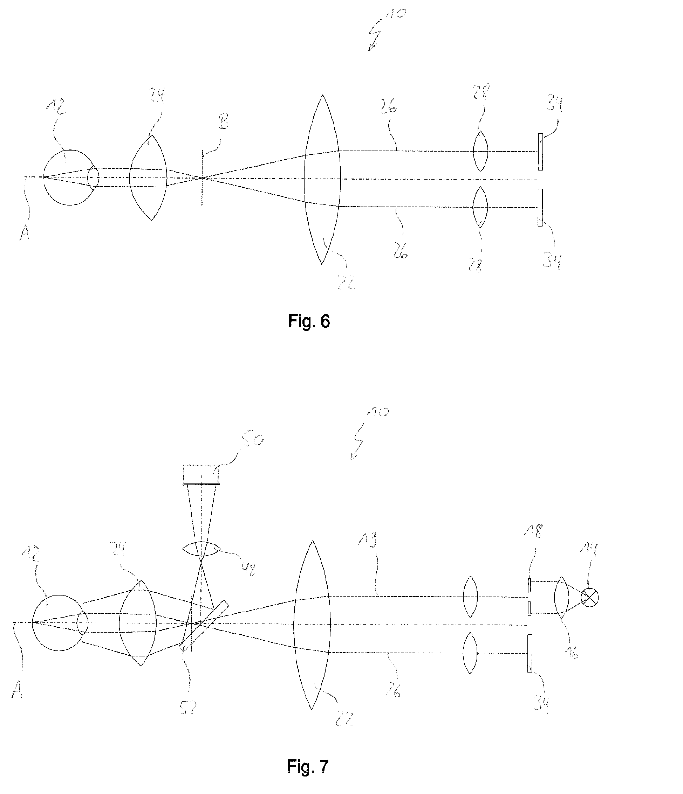

Figur 6 zeigt eine schematische Darstellung einer weiteren Ausführungsform des erfindungsgemäßen Ophthalmoskops 10 mit zwei Beobachtungsstrahlen 26 für stereoskopische Abbildungen. Der Beleuchtungsstrahlengang liegt hierbei außerhalb der Zeichenebene und ist in Figur 6 nicht dargestellt. In dieser Ausführungsform sind zwei benachbarte elektronische Sensoren 34 vorgesehen, um stereoskopische Aufnahmen des Auges 12 anzufertigen. Der Beleuchtungsstrahlengang kann hierbei entweder wie in Figur 2 gewählt sein, d.h. mit einer Schlitzblende 18, die vor einer Beleuchtungsvorrichtung 14 hin- und her oszilliert, oder wie in Figur 5 gezeigt, d.h. mit einer Linienfokussieroptik im Beleuchtungsstrahl 19. Die Vorteile der vorliegenden Erfindung, nämlich die Vermeidung mechanisch hin- und her oszillierender Schlitzblenden, kommen bei der Ausführungsform der Figur 6 besonders deutlich zum Tragen, denn bei entsprechenden Ophthalmoskopen 10 des Stands der Technik mit der Möglichkeit der stereoskopischen Beobachtung sind insgesamt drei hin- und her oszillierende Schlitzblenden erforderlich, nämlich eine Schlitzblende im Beleuchtungsstrahl 19 sowie zwei Schlitzblenden 32 in den beiden zueinander im wesentlichen parallelen Beobachtungsstrahlen. Dies führt gerade bei stereoskopischen Ophthalmoskopen 10 des Stands der Technik zu ausgeprägten Vibrationsproblemen, die durch die Erfindung vermieden werden.

Figur 7 zeigt eine weitere Ausführungsform des erfindungsgemäßen Ophthalmoskops 10 mit einer zusätzlichen Abbildungseinheit, die den Augenvordergrund durch die ophthalmoskopische Linse 24 und eine weitere Linse 48 auf einen separaten Bildsensor 50 abbildet. Beim separaten Bildsensor 50 handelt es sich vorzugsweise ebenfalls um einen elektronischen Sensor mit aktiven Pixeln, beispielsweise einen CMOS-Sensor, wie er vorstehend ausführlich beschrieben wurde.

Die Trennung des Strahlengangs von Augenhintergrund- und Augenvordergrundbeobachtung wird in dieser Ausführungsform durch einen Strahlteiler 52 vorgenommen, wobei vorzugsweise ein Folienstrahlteiler (pellicle beam splitter) zum Einsatz kommt.

Der Augenvordergrund wird in dieser Ausführungsform mit einer in Figur 7 nicht dargestellten separaten Lichtquelle beleuchtet, die direkt vor dem Auge 12 positioniert ist und idealerweise im Infraroten emittiert.

Ansonsten entspricht die Ausführungsform der Figur 7 jener der Figur 2, d.h. mit einer mechanisch oszillierenden Schlitzblende 18 im Beleuchtungsstrahl 19. Selbstverständlich kann jedoch auch bei der Ausführungsform der Figur 7 die anhand der Figur 5 erläuterte Linienfokussieroptik zum Einsatz gelangen.

Die Erfindung ist nicht auf die als nicht beschränkende Beispiele vorgestellten Ausführungsformen beschränkt. So versteht sich beispielsweise, dass der anhand der Figuren 3a bis 3c erläuterte elektronische Sensor 34 nicht nur die dort aus Gründen der Einfachheit gezeigten zehn Pixelzeilen aufweist, sondern in der Praxis mehr als tausend Pixelzeilen.

Ferner versteht sich, dass der konkrete Verlauf der Strahlengänge des Beleuchtungsstrahls 19 sowie des Beobachtungsstrahls 26 bzw. der Beobachtungsstrahlen 26 im Falle stereoskopischer Abbildungen durch bekannte optische Elemente variiert werden kann. Ebenso versteht es sich, dass weitere Lichtstrahlen in die vorgestellten Strahlengänge eingekoppelt werden können, beispielsweise zu Therapie- oder Diagnosezwecken, wie es aus dem Stand der Technik grundsätzlich bekannt ist.

In all diesen abgewandelten Ausführungsformen bietet die Erfindung nicht nur die oben erläuterten Vorteile einer deutlichen Verringerung bzw. vollständigen Beseitigung mechanischer Vibrationen aufgrund hin- und her schwingender Schlitzblenden, sondern zahlreiche weitere Vorteile:
a) Wie bereits betont wurde, befindet sich die elektronische Blende beim elektronischen Sensor 34 in dessen Bildebene, so dass die Ausblendung von Streulicht optimiert wird.
b) Kontinuierliche Bildgebungsverfahren setzen eine ausreichend hohe Bildwiederholungsfrequenz von mehr als 10Hz voraus, um einen flüssigen Bewegungseindruck beim Beobachter zu erzeugen. Die im Stand der Technik eingesetzten Schlitzblenden müssen also mit hoher Frequenz schwingen, was in der Praxis nur mit einem mechanischen Oszillator in Resonanz zu erreichen ist. Dies setzt wiederum eine exakte und auf geringe Abweichungen äußerst empfindliche Abstimmung der Eigenfrequenz des Oszillators mit der Bildwiederholungsfrequenz des CCD-Sensors 30 voraus. Somit ist die Bildwiederholfrequenz für einen gegebenen Oszillator fest. Beim erfindungsgemäßen Ophthalmoskop 10 hingegen, insbesondere in der Ausführungsform gemäß Figur 5, die ohne jegliche mechanische hin- und her schwingende Schlitzblende arbeitet, ist keine derartige Beschränkung durch eine Resonanzbedingung gegeben.
c) Beim Ophthalmoskop des Stands der Technik ist die Spaltbreite für eine gegebene mechanisch schwingende Blende unveränderlich. Viele Anwendungen verlangen jedoch nach einer feinstufigen Variation der Spaltbreite und damit der effektiven Belichtungszeit. Beispielsweise ist eine Variation der Schlitzbreite oder der Bildwiederholfrequenz und damit der effektiven Belichtungszeit wünschenswert, um auf unterschiedliche Anwendungsfälle einzugehen: bei Angiographie des Augenhintergrunds muß die schwache Fluoreszenzemission eines Farbstoffes detektiert werden, was eine möglichst lange Belichtungszeit erfordert. Dies ist durch Einstellen der Parameter des elektronischen Sensors 34, insbesondere die Parameter N, Δt und t_{INT}, problemlos möglich.
d) Beim Ophthalmoskop des Stands der Technik beträgt die Frequenz des Oszillators für den Antrieb der Schlitzblende in der Praxis ein Vielfaches der Bildwiederholfrequenz des CCD-Sensors, da mechanische Oszillatoren mit geringer Frequenz zum einen ungenau in ihrer Schwingfrequenz abzustimmen sind und zum anderen auch zu besonders unangenehmen Vibrationen des gesamten Gerätes führen. Dies führt zu Doppelbelichtungen auf dem CCD-Sensor 30 und damit zu Bildunschärfe und Bewegungsartefakten durch schnelle Bewegung des Auges 12 oder sonstigen untersuchten Objekts. Die Verwendung von Bildsensoren, die nach einem der gängigen Video-Standards (PAL, NTSC) zwei Halbbilder, die zu unterschiedlichen Zeitpunkten aufgenommen werden, zu einem Vollbild zusammenfügen (Interlacing), hat sich als besonders nachteilig erwiesen. Die weitere computergestützte Bearbeitung und Auswertung der Bilder (z.B. die Bestimmung von Lage, Größe, Form etc. von Blutgefäßen im Auge 12) wird durch derartige Bewegungsartefakte oder Interlacing-Artefakte erschwert. Die erfindungsgemäße Vermeidung der mechanischen Schlitzblende vor dem Sensor löst all diese Probleme.
e) Der in Resonanz schwingende mechanische Oszillator zum Antrieb der Schlitzblenden beim Ophthalmoskop des Stands der Technik führt in der Regel eine sinusförmige Bewegung aus. Die integral während einer Periode durch die schwingende Blende auf das Auge 12 fallende Lichtmenge ist daher ungleichmäßig über das Auge 12 verteilt und führt zu einer ungleichmäßigen Kontrast- und Helligkeitsverteilung im Bild. Um dennoch eine gleichmäßige Ausleuchtung des Bildausschnitts zu erhalten, wird die Amplitude der Schwingung häufig so weit erhöht, dass nur der näherungsweise lineare Anteil der Blendentrajektorie im Sichtfeld liegt. Dadurch wird jedoch nur ein Bruchteil des auf das Auge 12 fallenden Lichts zur Bildgebung benutzt, was zu einer Verringerung der Sensitivität des Ophthalmoskops 10 führt. Beim erfindungsgemäßen Ophthalmoskop 10 hingegen wird das verfügbare Licht wesentlich besser ausgenutzt.
f) Durch die Verwendung einer herkömmlichen Glühlampe oder Halogenlampe, die die Blendenebene des Beleuchtungsstrahlenganges gleichmäßig ausleuchtet, wird die verfügbare Leuchtkraft der Beleuchtungsvorrichtung 14 nur mangelhaft ausgenutzt. Die Wärmeabstrahlung der Beleuchtungsvorrichtung 14 führt zu einer weiteren Beeinträchtigung der Handhabung des Gerätes. Diese Probleme lassen sich bei der in Figur 5 gezeigten Ausführungsform mit einem Laser und einer Linienfokussieroptik vermeiden.
g) Eine Halogenlampe, wie sie im Stand der Technik eingesetzt wird, strahlt ein breites, thermisches Frequenzspektrum ab, dessen Maximum im Infrarotbereich liegt, und das zu kürzeren Wellenlängen hin abfällt. Eine solche Lichtquelle ist dafür ungeeignet, ein kontrastreiches Farbbild des Augenhintergrunds zu erzeugen, da der Augenhintergrund hauptsächlich im roten Spektralbereich reflektiert. Halbleiterbasierte Bildsensoren sind zudem stärker im roten und infraroten Spektralbereich empfindlich als bei kürzeren Wellenlängen. Ein mit Halogenbeleuchtung aufgenommenes Farbbild des Augenhintergrunds hat daher einen dominanten Rot-Kanal, jedoch nur schwach ausgesteuerte Grün- und Blau-Kanäle. Das Bild ist daher verrauscht und kontrastarm. Der Einsatz von Filtern zur Beeinflussung des Beleuchtungspektrums verbessert zwar den Farbeindruck, reduziert jedoch stark die Leuchtkraft der Lichtquelle. Für die bereits angesprochene Beobachtung von Fluoreszenzemissionen ist aber eine intensive Anregung der Farbstoffe in einem engen Spektralbereich notwendig. Dies können in der Ausführungsform gemäß Figur 5 moderne Lichtquellen wie z.B. LEDs oder Laser leisten.

## Patentansprüche

1. Ophthalmoskop (10) zur Untersuchung eines Auges (12) eines Patienten, umfassend:
eine Beleuchtungsvorrichtung (14) zur Erzeugung eines Beleuchtungsstrahls (19);
eine Beleuchtungs-Abbildungsoptik zum Abbilden des Beleuchtungsstrahls (19) auf das Auge (12), und
Mittel (18) zum Scannen des Beleuchtungsstrahls (19) über das Auge (12), sowie
eine Beobachtungsvorrichtung (30), die einen elektronischen Sensor (34) mit einem Feld von fotosensitiven Pixeln (36) umfaßt, die jeweils zeilenweise aktivierbar und/oder auslesbar sind;
eine Beobachtungs-Abbildungsoptik zum Abbilden eines durch Reflexion des Beleuchtungsstrahls (19) am Auge (12) erzeugten Beobachtungsstrahls auf die Beobachtungsvorrichtung (30), und
Mittel (32) zum Ausblenden von Streulicht aus dem Beobachtungsstrahl,
**dadurch gekennzeichnet, dass**
die Mittel (32) zum Ausblenden von Streulicht aus dem Beobachtungsstrahl eine elektronische Steuerschaltung zum zeilenweisen Aktivieren und Auslesen jeder Pixelzeile des Sensors umfassen, und dass das Ophthalmoskop (10) eine elektronische Kontrolleinheit (38) umfaßt, die dazu ausgelegt ist, die Mittel (18) zum Scannen und die elektronische Steuerschaltung derart zu kontrollieren, dass das Scannen des Beleuchtungsstrahls (19) über das Auge (12) synchron mit der Aktivierung von Pixelzeilen erfolgt.

2. Ophthalmoskop (10) nach Anspruch 1, **dadurch gekennzeichnet, dass** die elektronische Steuerschaltung dazu ausgelegt ist, jeweils eine einzelne Pixelzeile auszulesen.

3. Ophthalmoskop (10) nach Anspruch 2, **dadurch gekennzeichnet, dass** die elektronische Steuerschaltung dazu ausgelegt ist, jeweils nach Ablauf einer einstellbaren Zeitverzögerung (Δt) die aktuell auszulesende Pixelzeile zu ändern, insbesondere von einer Pixelzeile zu einer benachbarten Pixelzeile.

4. Ophthalmoskop (10) nach einem der Ansprüche 1 bis 3, **dadurch gekennzeichnet, dass** die elektronische Steuerschaltung ferner dazu ausgelegt ist, jede Pixelzeile während einer einstellbaren Belichtungszeit (t_{INT}) vor ihrem Auslesen zu aktivieren.

5. Ophthalmoskop (10) nach einem der Ansprüche 1 bis 4, **dadurch gekennzeichnet, dass** die elektronische Steuerschaltung dazu ausgelegt ist, den Auslesevorgang infolge eines externen Triggersignals zu starten.

6. Ophthalmoskop (10) nach einem der vorhergehenden Ansprüche; **dadurch gekennzeichnet, dass** die Mittel (18) zum Scannen des Beleuchtungsstrahls (19) über das Auge (12) einen schwenkbar gelagerten Spiegel (44) mit einer Scanvorrichtung umfassen.

7. Ophthalmoskop (10) nach Anspruch 6, **dadurch gekennzeichnet, dass** es ferner eine feste Spaltblende zum Auskoppeln eines linienförmigen Beleuchtungsstrahls (19) umfaßt.

8. Ophthalmoskop (10) nach Anspruch 6, **dadurch gekennzeichnet, dass** es ferner eine Linienfokussieroptik (40, 42) zum Fokussieren des Beleuchtungsstrahls (19) in einer Linie umfaßt.

9. Ophthalmoskop (10) nach Anspruch 8, **dadurch gekennzeichnet, dass** die Linienfokussieroptik (40, 42) eine Zylinderlinse (42) umfaßt.

10. Ophthalmoskop (10) nach einem der Ansprüche 6 bis 9, **dadurch gekennzeichnet, dass** die elektronische Kontrolleinheit (38) dazu ausgelegt ist, die elektronische Steuerschaltung des elektronischen Sensors (34) und die Scanvorrichtung des schwenkbaren Spiegels (44) zu kontrollieren.

11. Ophthalmoskop (10) nach Anspruch 10, **dadurch gekennzeichnet, dass** die Stärke der Linie des fokussierten Beleuchtungsstrahls (19) der Höhe einer Pixelzeile des elektronischen Sensors (34) entspricht.

12. Ophthalmoskop (10) nach einem der Ansprüche 8 bis 11, **dadurch gekennzeichnet, dass** die Beleuchtungsvorrichtung (14) ein Laser ist.

13. Ophthalmoskop (10) nach einem der vorhergehenden Ansprüche, **dadurch gekennzeichnet, dass** die Beobachtungsvorrichtung (30) zwei elektronische Sensoren (34) zur stereoskopischen Untersuchung des Auges (12) umfaßt.

14. Ophthalmoskop (10) nach einem der vorhergenden Ansprüche, **dadurch gekennzeichnet, dass** es ferner eine separate Lichtquelle und einen separaten Bildsensor (50) zur Untersuchung des Auges (12) umfaßt.

## Claims

1. Ophthalmoscope (10) for examining a patient's eye (12), comprising:
an illumination device (14) for generating an illumination beam (19);
illumination imaging optics for imaging the illumination beam (19) onto the eye (12), and
means (18) for scanning the illumination beam (19) over the eye (12), as well as
an observation device (30) which comprises an electronic sensor (34) with an array of photosensitive pixels (36), which can respectively be activated and/or read out in rows;
observation imaging optics for imaging an observation beam, generated by reflection of the illumination beam (19) from the eye (12), onto the observation device (30), and
means (32) for excluding stray light from the observation beam,
**characterised in that**
the means (32) for excluding stray light from the observation beam comprise an electronic drive circuit for row-by-row activation and readout of each pixel row of the sensor, and **in that** the ophthalmoscope (10) comprises an electronic control unit (38) which is adapted to control the scanning means (18) and the electronic drive circuit so that the scanning of the illumination beam (19) over the eye (12) is synchronous with the activation of pixel rows.

2. Ophthalmoscope (10) according to Claim 1, **characterised in that** the electronic drive circuit is adapted respectively to read out a single pixel row.

3. Ophthalmoscope (10) according to Claim 2, **characterised in that** the electronic drive circuit is adapted to change the current pixel row to be read out, in particular from one pixel row to a neighbouring pixel row, respectively after an adjustable time delay (Δt) has elapsed.

4. Ophthalmoscope (10) according to one of Claims 1 to 3, **characterised in that** the electronic drive circuit is furthermore adapted to activate each pixel row for an adjustable exposure time (t_{INT}) before it is read out.

5. Ophthalmoscope (10) according to one of Claims 1 to 4, **characterised in that** the electronic drive circuit is adapted to start the readout process in response to an external trigger signal.

6. Ophthalmoscope (10) according to one of the preceding claims, **characterised in that** the means (18) for scanning the illumination beam (19) over the eye (12) comprise a tiltably mounted mirror (44) with a scanning device.

7. Ophthalmoscope (10) according to Claim 6, **characterised in that** it furthermore comprises a fixed slit shutter for extracting a linear illumination beam (19).

8. Ophthalmoscope (10) according to Claim 6, **characterised in that** it furthermore comprises line focusing optics (40, 42) for focusing the illumination beam (19) in a line.

9. Ophthalmoscope (10) according to Claim 8, **characterised in that** the line focusing optics (40, 42) comprise a cylinder lens (42).

10. Ophthalmoscope (10) according to one of Claims 6 to 9, **characterised in that** the electronic control unit (38) is adapted to control the electronic drive circuit of the electronic sensor (34) and the scanning device of the tiltable mirror (44).

11. Ophthalmoscope (10) according to Claim 10, **characterised in that** the thickness of the line of the focused illumination beam (19) corresponds to the height of a pixel row of the electronic sensor (34).

12. Ophthalmoscope (10) according to one of Claims 8 to 11, **characterised in that** the illumination device (14) is a laser.

13. Ophthalmoscope (10) according to one of the preceding claims, **characterised in that** the observation device (30) comprises two electronic sensors (34) for stereoscopic examination of the eye (12).

14. Ophthalmoscope (10) according to one of the preceding claims, **characterised in that** it furthermore comprises a separate light source and a separate image sensor (50) for examining the eye (12).

## Revendications

1. Ophtalmoscope (10) pour l'examen d'un oeil (12) d'un patient, comprenant :
un dispositif d'éclairage (14) pour produire un faisceau d'éclairage (19) ;
une optique de projection d'éclairage pour projeter le faisceau d'éclairage (19) sur l'oeil (12) et
des moyens (18) pour réaliser un balayage du faisceau d'éclairage (19) sur l'oeil (12), ainsi qu'
un dispositif d'observation (30), qui comprend un capteur électronique (34) comportant un champ de pixels photosensibles (36) qui peuvent respectivement être activés et/ou lus ligne par ligne ;
une optique de projection d'observation pour projeter sur le dispositif d'observation (30) un faisceau d'observation produit par réflexion du faisceau d'éclairage (19) sur l'oeil (12),
des moyens (32) pour éliminer la lumière diffusée provenant du faisceau d'observation,
**caractérisé en ce que**
les moyens (32) d'élimination de la lumière diffusée provenant du faisceau d'observation comprennent un circuit électronique de commande pour activer et lire ligne par ligne chaque ligne de pixels du capteur, et **en ce que** l'ophtalmoscope (10) comprend une unité électronique de contrôle (38) agencée pour contrôler les moyens (18) de balayage et le circuit électronique de commande d'une manière telle que le balayage du faisceau d'éclairage (19) sur l'oeil (12) ait lieu d'une manière synchrone à l'activation de lignes de pixels.

2. Ophtalmoscope (10) suivant la revendication 1, **caractérisé en ce que** le circuit électronique de commande est agencé de façon à chaque fois lire une ligne de pixels unique.

3. Ophtalmoscope (10) suivant la revendication 2, **caractérisé en ce que** le circuit électronique de commande est agencé de façon à chaque fois remplacer la ligne de pixels actuellement en lecture, notamment en passant d'une ligne de pixels à une ligne de pixels voisine, après écoulement d'un temps de retard (Δt) réglable.

4. Ophtalmoscope (10) suivant l'une des revendications 1 à 3, **caractérisé en ce que** le circuit électronique de commande est en outre agencé de façon à activer chaque ligne de pixels, avant sa lecture, pendant un temps d'exposition (t_{INT}) réglable.

5. Ophtalmoscope (10) suivant l'une des revendications 1 à 4, **caractérisé en ce que** le circuit électronique de commande est agencé de façon à démarrer l'opération de lecture sous l'effet d'un signal de déclenchement extérieur.

6. Ophtalmoscope (10) suivant l'une des revendications précédentes, **caractérisé en ce que** les moyens (18) de balayage du faisceau d'éclairage (19) sur l'oeil (12) comprennent un miroir (44) monté pivotant qui comporte un dispositif de balayage.

7. Ophtalmoscope (10) suivant la revendication 6, **caractérisé en ce qu'**il comprend en outre un diaphragme à fente pour le découplage d'un faisceau d'éclairage (19) en forme de ligne.

8. Ophtalmoscope (10) suivant la revendication 6, **caractérisé en ce qu'**il comprend en outre une optique de focalisation de ligne (40, 42) pour focaliser le faisceau d'éclairage (19) suivant une ligne.

9. Ophtalmoscope (10) suivant la revendication 8, **caractérisé en ce que** l'optique de focalisation de ligne (40, 42) comprend une lentille cylindrique (42).

10. Ophtalmoscope (10) suivant l'une des revendications 6 à 9, **caractérisé en ce que** l'unité électronique de contrôle (38) est agencée de façon à contrôler le circuit électronique de commande du capteur électronique (34) et le dispositif de balayage du miroir pivotant (44).

11. Ophtalmoscope (10) suivant la revendication 10, **caractérisé en ce que** l'épaisseur de la ligne du faisceau d'éclairage (19) focalisé correspond à la hauteur d'une ligne de pixels du capteur électronique (34).

12. Ophtalmoscope (10) suivant l'une des revendications 8 à 11, **caractérisé en ce que** le dispositif d'éclairage (14) est un laser.

13. Ophtalmoscope (10) suivant l'une des revendications précédentes, **caractérisé en ce que** le dispositif d'observation (30) comprend deux capteurs électroniques (34) pour l'examen stéréoscopique de l'oeil (12).

14. Ophtalmoscope (10) suivant l'une des revendications précédentes, **caractérisé en ce qu'**il comprend en outre une source séparée de lumière et un capteur d'image (50) séparé pour l'examen de l'oeil (12).
